# EUROPEAN PATENT SPECIFICATION

(11) **EP 4 531 965 B1**
(45) Date of publication and mention of the grant of the patent: **29.04.2026**
(21) Application number: 23734813.1
(22) Date of filing: 31.05.2023
(51) Int. Cl.: A61M 11/00, A61M 15/08, A61M 15/00

(54) **NASAL SPRAY GUIDE**
NASENSPRAYFÜHRUNG
GUIDE DE PULVÉRISATION NASALE

(30) Priority: 31.05.2022 US 202217829286
(43) Date of publication of application: 09.04.2025
(60) Divisional application: 26167125.9
(73) Proprietor: Oyster Point Pharma, Inc., Morgantown, West Virginia 26505 (US)
(72) Inventor: LOKHNAUTH, John, K., Princeton, NJ 08540 (US); DONATO, George, J., Princeton, NJ 08540 (US); NAU, Jeffrey, Alan, Pennington, New Jersey 08534 (US)
(74) Representative: Gill Jennings & Every LLP
(86) International application number: PCT/US2023/023936
(87) International publication number: WO 2023/235359

(56) References cited:
- WO-A1-2020/197995
- WO-A1-2021/066195
- WO-A1-98/57690
- WO-A2-03/095006

## Description

### CROSS-RELATED APPLICATIONS

This application claims priority to and is a continuation of United States Patent Application No. 17/829,286, filed May 31, 2022.

### TECHNICAL FIELD

This invention relates generally to the field of nasal spray devices.

### BACKGROUND

Many substances, such as fluid medications, may be delivered intranasally with a nasal spray device. A typical nasal spray device includes a nasal spray tip and a pump coupled to a container that holds fluid to be delivered via nasal spray. A user may insert the nasal spray tip into his or her nostril, then actuate the pump in order to drive fluid from the container to the nasal spray tip, which delivers the fluid in the form of a fine mist within the nostril.

It is often important that the fluid be delivered to a certain intended tissue target (e.g., an area with high vascularization for improved absorption). The location of drug delivery can be affected, for example, by the spray pattern, droplet size distribution, and specific plume geometry of the fluid distributed by the nasal spray tip, and/or influenced by user behavior in positioning the nasal spray device when actuating the pump. While device manufacturers have some degree of control over the spray characteristics through design considerations, they have little to no control in preventing improper operation of the device by a user. For example, when using a conventional nasal spray device, a user may insert the nasal spray tip too far into the nostril, which may result in administered fluid being delivered to the back of the throat (e.g., post-nasal drop) instead of being delivered to desired nasal tissue. As another example, some drugs are intended to be delivered toward one side (e.g., lateral side) of the nostril, but users may incorrectly and/or inconsistently angle the nasal spray tip, which may lead to inaccurate drug administration.

Thus, there is a need for additional systems and methods for guiding delivery of a substance via nasal spray.

WO 2020/197995 A1 discloses medical devices and methods, particularly systems and methods for delivering medication to a patient's nasal cavity.

WO 2021/066195 A1 discloses a nasal spray/spout nozzle for administering a medicament into a nasal cavity and also discloses a nasal rest article for such administration of the medicament.

WO 03/095006 A2 discloses a fluid dispensing device for use as a nasal inhaler.

### SUMMARY

A nasal spray guide may be used in conjunction with a nasal spray device to assist administration of a fluid from the nasal spray device. Generally, the nasal spray guide of the invention includes a collar defining a lumen configured to receive a nasal spray tip, and a shoulder extending radially outward from the collar, where the shoulder comprises a shoulder surface angled relative to the lumen and an inner surface of the collar comprises one or more radially inward projections. In some variations, an inner diameter of the lumen may vary.

In some variations, the shoulder surface may be obliquely angled relative to the lumen, such as at an angle between about 60 degrees and about 80 degrees (e.g., about 75 degrees). The shoulder surface may surround at least a portion of the lumen, and in some variations may substantially surround the lumen. In some variations, the shoulder surface may be substantially planar and elliptical, though the shoulder surface may have any suitable degree of out-of-plane curvature (e.g., concave, convex) and/or shape (e.g., circular, rectangular, etc.).

In some variations, the nasal spray guide may further include a cap defining a cavity and configured to engage the shoulder such that the cavity and the lumen are substantially aligned. The cap may, for example, cover and/or enclose a nasal spray tip within the cavity, such as between administrations of fluid from the nasal spray device. In some variations, the cap may be standalone and separate from the collar and shoulder of the nasal spray guide, while in some variations the cap may be coupled to the collar and/or shoulder of the nasal spray guide. For example, in some variations the cap may be coupled to the collar, the shoulder, or both by a tether (e.g., flexible member).

Furthermore, in some variations the nasal spray guide may include an alignment indicator configured to indicate a desired predetermined orientation of the nasal spray guide with an anatomical landmark of the user. The alignment indicator may, for example, be located on the shoulder (e.g., shoulder surface) and/or collar of the nasal spray guide. The alignment indicator may have any suitable structure (e.g., a molded feature, a surface-applied label, a color-coded material, etc.) and/or communicate the alignment information in any suitable manner (e.g., graphic, text, color code, etc.). In an example variation, the nasal spray guide may include an alignment indicator in the form of an arrow molded on the shoulder surface, and may be configured to indicate a side of the shoulder that should be closer to a septum of the user, such that the shoulder surface is angled away from the septum.

Generally, in some variations, a method of assisting administration of fluid from a nasal spray device may include providing a nasal spray guide comprising a collar defining a lumen, and a shoulder extending radially outward from the collar, positioning at least a portion of the nasal spray device in the lumen such that a distal portion of the nasal spray device is exposed, inserting the distal portion of the nasal spray device in a nostril of a user until the shoulder of the nasal spray guide is seated against a nasal surface of the user, and actuating the nasal spray device while the shoulder of the nasal spray guide is seated against the nasal surface of the user. In some variations, the shoulder surface is obliquely angled relative to the lumen. In some variations (e.g., where administration of the fluid toward a target region comprising the inferior turbinate is desired), the method may include orienting the nasal spray guide such that the shoulder surface is angled away from a nasal septum of the user. The nasal spray guide may, in some variations, include an alignment indicator and orienting the nasal spray guide may include aligning the alignment indicator with an anatomical landmark on the user.

In some variations, the method may further include removing the nasal spray tip from the nostril of the user and covering the nasal spray tip with a cap. The cap may, in some variations, be coupled to the collar and/or the shoulder by a tether (e.g., flexible member), or may be standalone and separate from the rest of the nasal spray guide.

### BRIEF DESCRIPTION OF THE DRAWINGS

FIGS. 1A-1C depict illustrative schematics of a side view, a top plan view, and another side view, respectively, of an example variation of a nasal spray guide. FIG. 1D depicts an illustrative schematic of an example variation of a nasal spray guide coupled to a nasal spray device.
FIGS. 2A-2D depict illustrative schematics of example variations of a nasal spray guide with a cap. FIGS. 2E and 2F depict illustrative schematics of example variations of a nasal spray guide comprising multiple collar portions and/or shoulder portions.
FIG. 3 depicts an illustrative schematic of an example variation of a nasal spray guide with a wedge-shaped shoulder.
FIGS. 4A-4E depict illustrative schematics of example variations of a shoulder in a nasal spray guide.
FIGS. 5A and 5B depict illustrative schematics of example variations of a nasal spray guide with a cap.
FIGS. 6A and 6B depict illustrative schematics of an example variation of a nasal spray guide in use with a nasal spray device.
FIGS. 7A and 7B depict illustrative schematics of an example variation of a nasal spray guide.
FIGS. 8A and 8B depict illustrative schematics of an example variation of a nasal spray guide.
FIGS. 9A-9E depict illustrative schematics of an example variation of a nasal spray guide.
FIGS. 10A-10E depict illustrative schematics of an example variation of a nasal spray guide.
FIG. 11 depicts the nasal spray guide shown in FIGS. 10A-10E coupled to a nasal spray device.

### DETAILED DESCRIPTION

Non-limiting examples of various aspects and variations of the invention are described herein and illustrated in the accompanying drawings.

Described herein are devices and methods for assisting nasal spray delivery with a nasal spray device. For example, a nasal spray guide may be coupled to a nasal spray device and configured to guide and/or train a user to appropriately position a nasal spray device in his or her nose for improved delivery via the nasal spray device.

The nasal spray guide includes a collar defining a lumen configured to receive a nasal spray tip of a nasal spray device, and a shoulder extending radially outward from the collar. The lumen of the nasal spray guide may be sized so as to allow a distal end of a nasal spray tip (e.g., portion having a nozzle outlet) to extend beyond the collar. This arrangement may ensure that a distal end of the nasal spray tip (or at least the nozzle outlet of the nasal spray tip) remains exposed, thereby helping to prevent the nasal spray guide from interfering with or otherwise altering the nasal spray plume itself, thereby preserving the nasal spray distribution inherent in the nasal spray device. Generally, when the combination of the nasal spray guide and nasal spray device is in use, a surface of the shoulder of the nasal spray guide may be configured to be seated against the nose of the user and function as a depth limiter to help prevent excessive insertion of the nasal spray tip into a nostril. Furthermore, a surface of the shoulder is angled relative to the lumen (e.g., obliquely angled), so as to help guide positioning of the nasal spray tip within the nostril of the user at a desired angle or orientation to target fluid delivery toward a particular desired target tissue region.

In some variations, the nasal spray guide may be removably coupled to the nasal spray device. The nasal spray guide may be removed between uses (e.g., for washing or sterilization of the nasal spray guide, or of the nasal spray tip), or the nasal spray guide may be placed and retained on the nasal spray device between uses of the nasal spray device. The nasal spray guide may include (or be configured to operate with) a removable cap that covers the exposed portion of the nasal spray tip between uses. Alternatively, in some variations, the nasal spray guide or a portion thereof may be integrated with a nasal spray device.

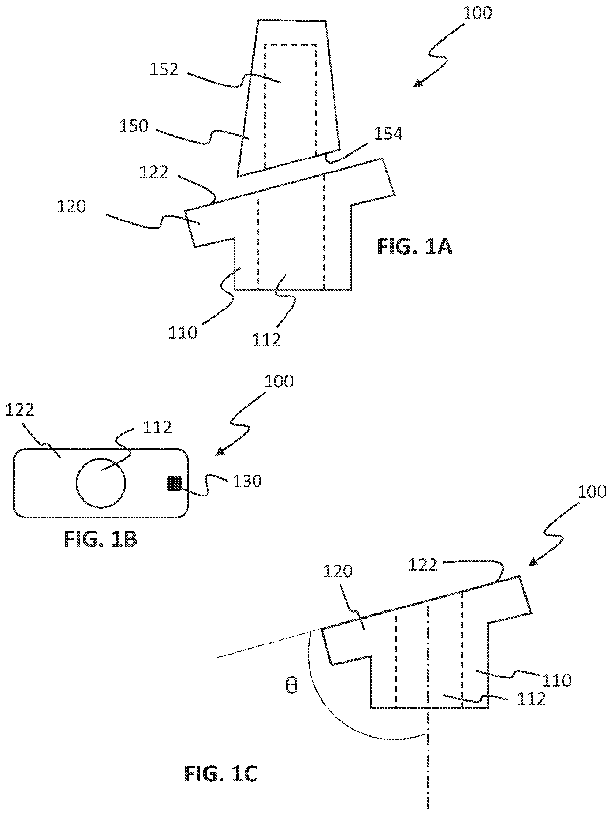

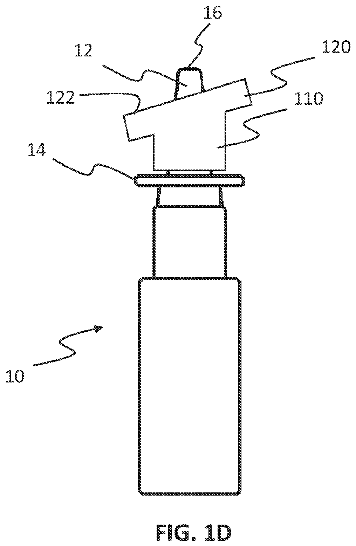

An example variation of a nasal spray guide 100 is shown in FIGS. 1A-1D. The nasal spray guide 100 may include a collar 110 defining a lumen 112 configured to receive a nasal spray tip, and a shoulder 120 extending radially outward from the collar 110. As shown in FIG. 1B, the shoulder 120 may at least partially surround the lumen 112, such that when a nasal spray tip of a nasal spray device 10 is inserted in the lumen 112 (as shown in FIG. 1D), the shoulder 120 at least partially surrounds the inserted nasal spray tip 12.

The nasal spray guide 100 may be placed onto a nasal spray tip 12 of a nasal spray device 10 in a manner that permits, and does not obstruct, the actuation of the nasal spray device 10. For example, as shown in FIG. 1D, the collar 110 may be placed around the nasal spray tip 12 and on the nasal spray device 10. The collar 110 may, in some variations, be seated on or adjacent a pump actuating surface 14 that is designed for actuation by a user's fingers to operate the nasal spray pump of the nasal spray device 10. In this configuration, the collar 110 may have a sufficient standoff height so as to provide enough clearance for a user's fingers to fit in the space between the pump actuating surface 14 and the shoulder 120. For example, at least one side of the collar 110 may have a standoff height that is at least about 0.5 cm, at least about 0.75 cm, at least about 1 cm, at least about 2 cm, at least 2.5 cm, or at least 3 cm, etc. Additionally or alternatively, the diameter of the portion of the collar 110 adjacent the pump actuating surface 14 may be sufficiently smaller than the diameter of the pump actuating surface 14 so as to leave enough surface area of the pump actuating surface 14 for a user's fingers to manipulate the pump (e.g., at least about 1.5 cm radial distance on either side of the collar 110 that is not covered by the nasal spray guide). For example, the outer diameter of collar 110 adjacent the pump actuating surface 14 (e.g., lower portion of collar 110, as shown in the orientation of FIG. 1D) may be between about 0.5 cm and about 2 cm, or between about 0.75 cm and about 1.75 cm, or between about 1 cm and about 1.5 cm, etc. Although example ranges for certain dimensions of the collar 110 are provided above, it should be understood that the collar 110 may have any suitable dimensions, depending on, for example, the dimensions of the nasal spray device with which the nasal spray guide 100 is to be used, and/or the user type (e.g., adult with larger fingers, child with smaller fingers). In some variations, the collar 110 may be provided in a kit, as one of various predetermined sizes (e.g., extra small, small, medium, large, extra large, etc.) or size ranges. The predetermined sizes may, for example, correspond to nose sizes and/or nasal spray pump sizes.

Furthermore, the nasal spray guide 100 may be placed onto a nasal spray tip 12 of a nasal spray device 10 such that it does not interfere with the distribution of the fluid sprayed from the nasal spray tip 12. For example, as shown in FIG. 1D, the collar 110 may be placed around the nasal spray tip 12, and the height of the collar 110 and shoulder 120 may be sized such that the distal end of the nasal spray tip 12 (e.g., including at least nasal spray outlet 16) extends beyond the shoulder 120 in an exposed manner. Since the uncovered nasal spray outlet 16 is not obstructed by the nasal spray guide 100, the nasal spray device 10 is permitted to release a nasal spray plume that is unaltered by the nasal spray guide, thereby maintaining the spray characteristics provided inherently by the design of the nasal spray device 10.

The collar 110 may function to attach the nasal spray guide to a nasal spray device, and/or function as a standoff to help determine the axial location of the nasal spray guide along the nasal spray tip and/or depth of permitted nasal spray tip insertion. For example, the height of the collar 110 (in combination with, for example, the thickness of the shoulder 120) may affect the location of the shoulder 120 relative to the nasal spray tip, and thus the depth at which the nasal spray tip may be inserted into a user's nostril.

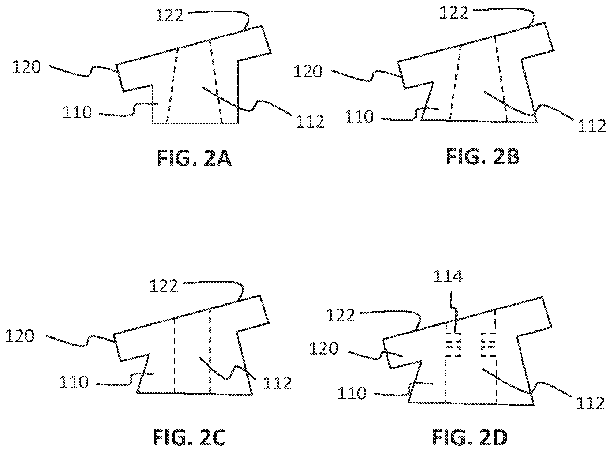

The collar 110 may have any suitable shape. For example, the nasal spray guide shown in FIG. 1A includes a generally cylindrical collar 110. However, in other variations the nasal spray guide may have any suitable cross-section, such as elliptical, triangular, square, rectangular, pentagonal, hexagonal, septagonal, octagonal, or any suitable number of sides. In some variations, the collar 110 may have a substantially constant cross-section along its length (e.g., prismatic or columnar with any suitable cross-section). In other variations, the collar 110 may have a varying cross-section along its length. For example, the collar 110 may be tapered as shown in FIGS. 2B-2D (e.g., frustoconical).

Furthermore, the lumen 112 defined in the collar 110 may have any suitable shape, such as with a cross-section that is circular, elliptical, etc. Additionally or alternatively, like the collar 110, the lumen 112 may have a substantially constant cross-section along its length passing from one end of the collar 110 to an opposite end of the collar 110 (e.g., as shown in FIG. 1A) or a varying cross-section along its length. For example, a lumen 112 may vary in cross-section by being tapered or frustoconical (e.g., as shown in FIGS. 2A and 2B). As another example, as shown in FIG. 2D, a lumen 112 includes one or more internal radial projections 114 or other inwardly oriented features (e.g., ridges) that result in an inner diameter of the collar that provides a lumen that has a reduced diameter in the region of the internal radial projections 114, or otherwise varies in size. In some variations, a varied diameter of the lumen may be configured to match the tapered dimensions of a nasal spray tip intended to be inserted into the lumen 112, so as to be compatible with a specific kind of nasal spray tip (e.g., brand or size of nasal spray bottle). Maintaining a close fit between the lumen and the nasal spray tip may, for example, help lock or retain the nasal spray guide onto the nasal spray device.

In some variations, the diameter of the lumen 112 may be adjustable in order to accommodate a variety of nasal spray tips sizes and/or shapes. For example, the inner surface of the lumen 112 may include or be lined with a conformable material (e.g., silicone or other elastomer) that can compress to accommodate a range of nasal spray tip sizes and/or shapes. Such conformable material may be located along the entire internal surface of the lumen 112, or along only a portion of the internal surface of the lumen 112 (e.g., conformable rings). As another example of an adjustable lumen 112, the collar 110 may include one or more circumferentially-distributed radially flexing members that are biased inward but flex outward to adjust to receive and clamp around a nasal spray tip 12 therebetween. Such radially flexing members may, for example, be circumferential or arcuate flexing members extending laterally around the lumen (e.g., spiral-like arms), or longitudinal flexing members extending axially along the lumen.

Although a close fit between the lumen 112 and the nasal spray tip 12 received in the nasal spray guide 100 may help retain the nasal spray guide onto the nasal spray device 10 as described above, additionally or alternatively the nasal spray guide 100 may include one or more engagement features to help couple the nasal spray guide to the nasal spray device 10. For example, the nasal spray device 10 may be configured to snap fit over the nasal spray tip (e.g., via interlocking engagement features on the collar 110 and a portion of the nasal spray device 10 such as the nasal spray tip 12 or pump actuation surface 14). As another example, the nasal spray device 10 may fit over the nasal spray tip (e.g., in a slip fit) and be axially retained in place with an arcuate clip (e.g., C-shaped clip) placed over or adjacent to the nasal spray device 10. As another example, a frictional surface (e.g., textured surface, elastomer lining or gasket) on the internal surface of the lumen 112 may help engage and/or retain the nasal spray guide over the nasal spray tip.

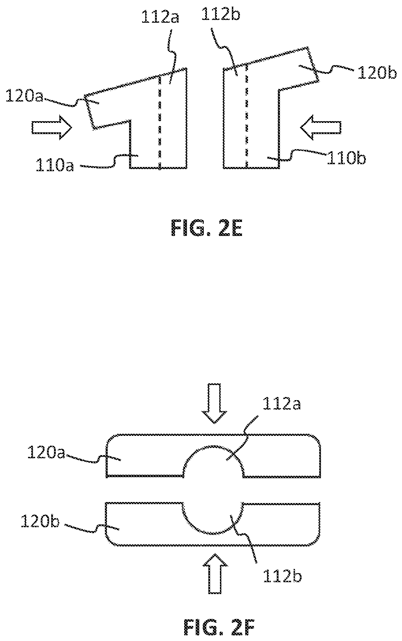

In some variations, the collar 110 may include a singular, ring-like piece defining a lumen therethrough. However, the collar 110 may alternatively include multiple collar portions that may be adjustable to form a lumen. For example, as shown in FIG. 2E, in an example variation the collar may include two longitudinal collar portions 110a and 110b. Collar portion 110a may include a respective shoulder portion 120a and semi-circular cutout 112a, and collar portion 110b may include a respective shoulder portion 120b and semi-circular cutout 112b. These collar portions 110a and 110b may be combined around a nasal spray tip to form a circular lumen from the respective semi-circular cutouts. FIG. 2F illustrates another example variation in which the collar includes two longitudinal collar portions (with respective shoulder portions 120a and 120b and semi-circular cutouts 112a and 112b) that are configured to similarly combine along a different axis than the variation shown in FIG. 2E. Alternatively, the collar 110 may include any suitable number of portions (e.g., three, four, etc.) that, when combined, form a lumen to receive a nasal spray tip. The collar portions may be equally sized (e.g., mirrored halves) or unequally sized. In some variations, the collar portions may be separately formed and connected via a suitable connector (e.g., hinge, ball and socket, etc.), though in other variations at least some of the collar portions may be integrally formed and adjustable relative to each other via a suitable connector-like member (e.g., living hinge). The multiple collar portions may be locked together around a nasal spray tip with any suitable engagement features, such as those described above.

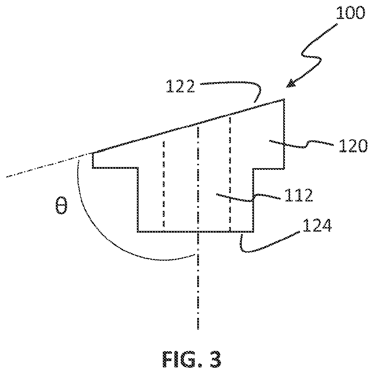

As described above, the shoulder 120 may function as a depth limiter and/or a guide for positioning the nasal spray tip relative to a user's nostril. The shoulder 120 may be integrally formed with the collar 110, or the shoulder 120 may be separately formed from and subsequently coupled to the collar 110 (e.g., with one or more fasteners, epoxy, mechanical interfit features, etc.). The shoulder 120 may include a shoulder surface 122 that is configured to contact the nose of the user and may be angled relative to the lumen 112 of the collar 110. In some variations, the shoulder surface 122 may be obliquely angled relative to the lumen 112, such as to help guide overall directional thrust of the nasal spray from the nasal spray tip, when the shoulder surface 122 is placed against the nose. For example, as shown in FIG. 1C, the shoulder surface 122 may be angled relative to the lumen 112 by an angle θ. In some variations, the angle θ may be between about 40 degrees and about 90 degrees, or between 60 degrees and about 80 degrees, or between about 70 degrees and about 80 degrees. In an example variation in which the nasal spray guide 100 is configured to help guide distribution of a nasal spray (axially from the distal tip of the nasal spray tip) toward the inferior turbinate when the nasal spray tip is inserted in a nostril, the angle θ may be about 75 degrees. As shown in FIG. 1C, the entire shoulder 120 may be angled at angle θ. However, in some variations, only a portion of the shoulder including the shoulder surface 122 may be angled at angle θ. For example, as shown in FIG. 3, the shoulder 120 may be wedge-shaped, with a first shoulder surface 122 that is obliquely angled relative to the lumen 112 at an angle θ, and a second shoulder surface 124 opposite the first shoulder surface 122 that is orthogonal to the lumen 112.

Furthermore, although the shoulder surface 122 is generally shown in FIG. 1A as substantially planar, it should be understood that in some variations, the shoulder surface 122 may be concave or convex. For example, the shoulder surface 122 may curve away from the collar 110 (e.g., configured to curve around a user's nose in use) or may curve towards the collar 110 (e.g., configured to curve away from a user's nose in use).

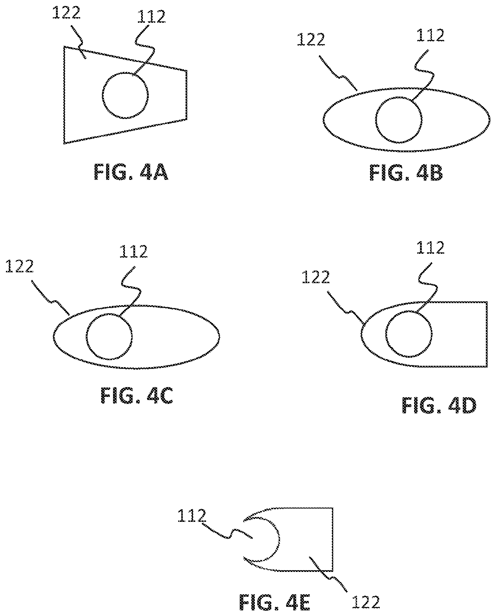

The shoulder surface 122 that is configured to be seated against or abut the user's nose may have any suitable overall shape. For example, as shown in FIG. 1B, the shoulder surface 122 may, as viewed from above, have a generally rectangular shape. However, the shoulder surface 122 may have any suitable polygonal shape with a suitable number of sides (e.g., 3, 4, 5, 6, or more), such as a square or a trapezoid (e.g., FIG. 4A). As another example, the shoulder surface 122 may be circular or elliptical (e.g., FIGS. 4B and 4C), or any combination of shapes such partially elliptical and partially polygonal (e.g., FIG. 4D).

Additionally, as described above, the shoulder surface 122 may be configured to at least partially surround the lumen 112 through which a nasal spray tip is inserted. In some variations, the lumen may be completely surrounded by the shoulder surface 122 (e.g., FIGS. 4A-4D). The lumen 112 may be positioned in the approximate center of the shoulder surface (e.g., FIGS. 4A and 4B) or may be offset from the center of the shoulder surface (e.g., FIGS. 4C and 4D) such that the lumen is closer to one end of the shoulder surface 122 than the other. Alternatively, in some variations the lumen 112 of the collar may be only partially surrounded by the shoulder surface 122 (e.g., FIG. 4E). Furthermore, similar to that described above with respect to nasal spray guides including multiple collar portions, in some variations the lumen 112 may be formed from multiple shoulder portions (e.g., as shown in FIGS. 2E and 2F).

As the shoulder surface 122 is configured to be seated against the nose of a user, the shoulder surface 122 may, in some variations, include one or more features to help maintain placement of the shoulder surface 122 against skin. For example, the shoulder surface 122 may include textural features (e.g., rim, ridges, channels, detents, raised dots, etc.) and/or a material that is conformable and/or frictional (e.g., silicone). Additionally or alternatively, the shoulder surface 122 may include one or more features to help improve user comfort when the shoulder surface 122 is placed against skin (e.g., conformable material, radiused edges, etc.).

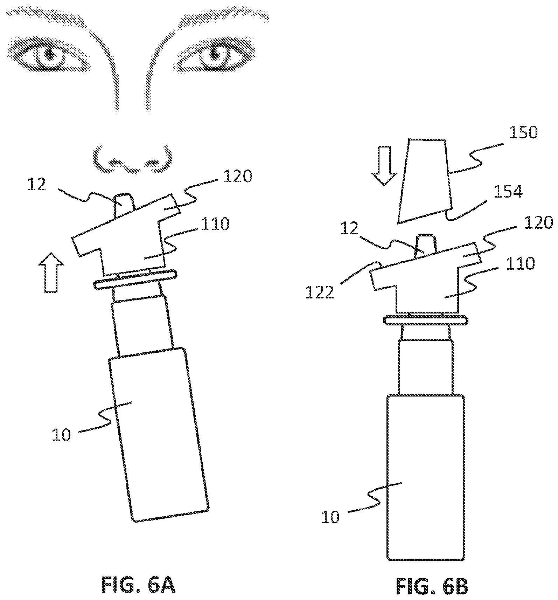

In some variations, the nasal spray guide 100 may include a cap 150 that functions to cover or otherwise enclose the nasal spray tip, such as between uses of the nasal spray device, to help keep the nasal spray tip clean when the nasal spray device is not in use. The cap 150 may define a cavity 152 and be configured to engage the shoulder 120 such that the cavity 152 and the lumen 112 are substantially aligned. As shown and described in further detail below, when the cap and the shoulder are engaged in such a manner, a nasal spray tip that has been inserted through the lumen 112 of the collar 110 may be received in the cavity 152 and thus covered by a closed end of the cap. For example, as shown in FIG. 6A, the cap 150 may be removed from the nasal spray tip 12 of a nasal spray device 10 when a user desires to use the nasal spray device 10 to administer fluid from the nasal spray device 10. Thereafter, the cap 150 may be returned to engage the shoulder 120 and cover the nasal spray tip 12 between such fluid administrations with the nasal spray device 10, as shown in FIG. 6B.

In some variations, the cavity 152 of the cap may have an open end that is at least partially surrounded by a cap surface 154 configured to abut the shoulder surface 122 when the cap 150 is placed over the nasal spray tip. As such, the cap surface 154 may be angled relative to the lumen 112 in a manner similar to the shoulder surface 122, and/or otherwise may be complementary to the shoulder surface 122 to provide a seal such that the nasal spray tip is enclosed between the cap 150 and the shoulder surface 122. Furthermore, in some variations the cap 150 may include one or more features to help align and/or secure the cap 150 against the shoulder surface 122. For example, the cap 150 and/or shoulder 120 may include one or more mechanical interfit features (e.g., snap-fit features) to help rotationally and/or laterally align the cavity 152 and lumen 112, and/or releasably lock the cap 150 and the shoulder 120 together. As another example, the cap 150 and/or shoulder 120 may include magnets or magnetic material to help rotationally and/or laterally align the cavity 152 and lumen 112, and/or releasably engage the cap 150 and the shoulder 120 together. The cap 150 may be configured to engage the shoulder 120 in an axial motion (e.g., up and down motion), or may engage via threaded features and/or other suitable separate fasteners.

As shown in FIG. 1A, in some variations the cap 150 may be generally tapered or frustoconical in shape. However, it should be understood that in other variations, the cap 150 may have any suitable shape or profile (e.g., cylindrical, prismatic, stepped profile, etc.). Additionally or alternatively, the outer surface of the cap 150 may have one or more features to improve grip and/or maneuverability of the cap 150. For example, in some variations the outer surface of the cap 150 may include one or more outwardly or inwardly expressed textural features such as a lip or flange, ring, bump, ridge, groove, and/or channel, and/or one or more frictional materials (e.g., rubber) to improve a user's ability to grasp the cap 150.

As shown in FIG. 1A, the cavity 152 may be generally cylindrical. However, it should be understood that in other variations, the cavity 152 may have any suitable shape or profile (e.g., tapered, frustoconical, prismatic, stepped profile, etc.). The overall dimensions (e.g., diameter, depth, etc.) and geometry may, for example, vary depending on the specific dimensions and features of the nasal spray tip that the cap 150 is intended to cover. In some variations, the cavity 152 may be sized and shaped to allow clearance (e.g., 1mm-2mm) between the nasal spray tip and an internal surface of the cap 150. Alternatively, in some variations the cavity 152 may be sized and shaped substantially similar to the exposed portion of the nasal spray tip, such that the cap 150 fits somewhat snugly over the nasal spray tip. Additionally or alternatively, in some variations the cavity 152 or other features of the cap 150 may have an adjustable size and/or shape to fit to a range of nasal spray tip types. For example, the cavity 152 may be lined with a conformable material, and/or the cap 150 may include an extendible material or construction (e.g., accordion pleats) to adjust to various lengths of nasal spray tips.

In some variations, the cap 150 may include one or more anti-infective substances or other features (e.g., anti-microbial, anti-fungal, etc.) to help reduce growth of pathogens on the nasal spray tip between uses. For example, the cap 150 may be impregnated with an anti-microbial material such as silver. As another example, the cap 150 may include an ultraviolet light source (e.g., UV-C) to selectively sanitize the nasal spray tip when the nasal spray tip is inserted in the cavity 152.

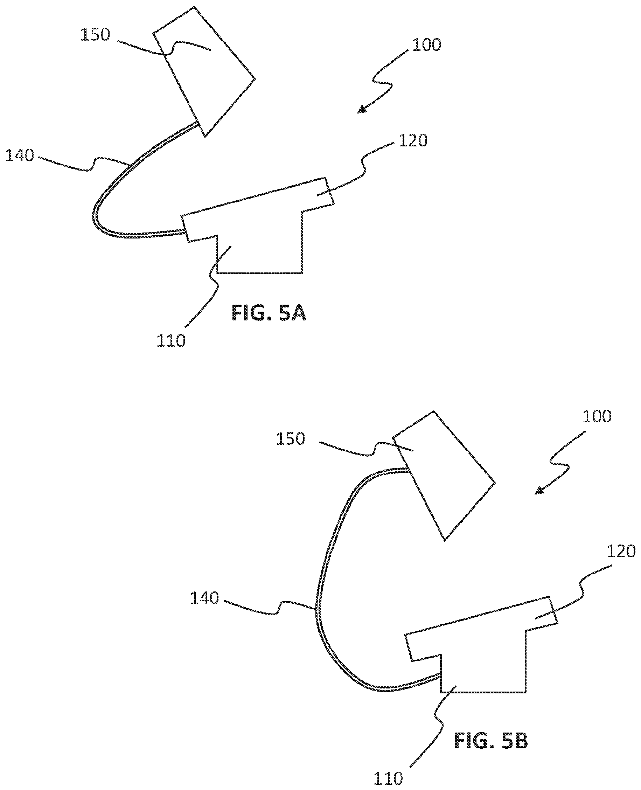

In some variations, the cap 150 may be separate from the rest of the nasal spray guide 100, as shown in FIG. 1A. However, in other variations, the cap 150 may be coupled to another portion of the nasal spray guide 100. For example, as shown in FIG. 5A, the cap 150 may be coupled to the shoulder 120. As another example, as shown in FIG. 5B, the cap 150 may be coupled to the collar 110. The cap 150 may be coupled to the rest of the nasal spray guide 100 via a tether 140, such as a living hinge or a flexible member extending between the cap 150 and the collar 110 and/or shoulder 120. The tether 140 may, for example, be integrally formed with the cap 150 and the collar 110 and/or shoulder 120 (e.g., via injection molding), or separately formed and coupled to the cap 150 and the collar 110 and/or shoulder 120 via one or more suitable fasteners (e.g., epoxy, mechanical connectors, snap fit features, etc.). The tether 140 may be permanent or removable. The length of the tether 140 may be long enough so as to not excessively constrain movement of the cap (e.g., enable the cap to be placed over and removed from the nasal spray tip). Thus, the length of the tether 140 may depend at least in part on the dimensions of the nasal spray tip and the relative locations of the attachment points of the ends of the tether140, though in some variations the length of the tether 140 may be at least 2 cm, at least 3 cm, at least 4 cm, or at least 5 cm or more.

In some variations, the tether 140 may be biased toward a predetermined shape such that the cap 150 is not aligned with the lumen 112 of the collar (e.g., a straight shape, arcuate shape, or other suitable shape biased toward leaving the nasal spray tip uncovered). Alternatively, in some variations the tether 140 may be biased toward a predetermined shape such that the cap 150 is aligned with the lumen 112 of the collar (e.g., an arcuate or other suitable shape biased toward leaving the nasal spray tip covered). In yet other variations, the tether may lack a bias toward any particular predetermined shape. For example, the tether may include a freely-movable string-like member, or may include a shapeable wire member that substantially retains the shape or form in which it is placed as the cap and tether are manipulated.

Although the nasal spray guide 100 is primarily described herein as including a cap 150, in some variations, the nasal spray guide 100 may omit a cap 150. For example, the nasal spray guide 100 may be configured for limited use (e.g., single use for one nostril, or two uses for left and right nostrils) and disposed after the limited use.

In some variations, the nasal spray guide may be intended to be oriented in a predetermined orientation relative to an anatomical landmark of the user. For example, the nasal spray guide may be intended to be oriented such that the angled shoulder surface (e.g., shoulder surface 122) helps position the nasal spray tip toward certain anatomy of the user's nose. In these variations, the nasal spray guide may include one or more alignment indicators 130 (e.g., as shown in FIG. 1B) to help a user orient the nasal spray guide in the predetermined orientation during use. An alignment indicator may be located on any suitable portion of the nasal spray guide, including the collar and/or shoulder (e.g., shoulder surface 122). Examples of alignment indicators include molded features, surface-applied labels, color-coded materials, other suitable marks, and/or the like. For example, in some variations the alignment indicator may include an arrow or other suitable graphic, a text label (e.g., "S" for septum), a pigmentation of shoulder or collar material (e.g., a differently-colored segment of the shoulder surface), etc. Furthermore, in some variations the shape of the shoulder and/or collar itself may help designate a predetermined orientation of the nasal spray guide. For example, the shoulder may have a teardrop-shaped or tapered shoulder surface that narrows in width such that the shoulder surface itself functions as an arrow-like alignment indicator. As another example, on a lateral side of the shoulder intended to be placed on a lateral side of the nose, the shoulder surface may include an outer rim or lip configured to curve upwards around the lateral side (e.g., ala) of the nose. Such an upwards curvature may help conform the overall shoulder surface 122 to the nose and discourage a user from mistakenly placing the outer rim or lip on a medial side of the nose near the septum, for example.

In one example variation, the nasal spray guide may be intended to be oriented such that an obliquely angled shoulder surface is angled away from a septum of the user, as shown in FIG. 6A. This may, for example, help guide a user in aiming a nasal spray tip 12 (and nasal spray plume distributed from the nasal spray tip) toward the lateral turbinate of a user. Accordingly, an alignment marker may be configured to designate a side of the shoulder surface that should be placed closest to the septum and farther from a lateral side of the nose (or alternatively, design a side of the shoulder surface that should be placed farthest from the septum and closer to a lateral side of the nose).

Some or all of the features of the nasal spray guide described herein may include a biocompatible rigid or semi-rigid material (e.g., suitable plastic) such that it sufficiently retains its outer shape and form to ensure its shoulder provides a suitable abutting surface to function as a depth limiter. The nasal spray guide 100 may, in some variations, be formed in any suitable manufacturing process, such as injection molding, casting, 3D printing, and/or suitable machining processes (e.g., turning). The nasal spray guide 100 may be constructed from a thermoplastic polymer that has a soft "rubber feel" and retains its shape at room temperature. In some variations, the thermoplastic polymer may comprise a styrene, an ethylene, a butylene or combinations thereof. In some variations, the thermoplastic polymer may be Everlon^{®} TM-42C, a styrene ethylene butylene styrene block copolymer. The thermoplastic polymer may comprise a antimicrobial agent, by way of example Withstand^{™} (Avient) to prevent accumulation of microbes.

To use the nasal spray guide, a user may position at least a portion of the nasal spray device (e.g., nasal spray tip 12) through the lumen 112 of the collar 110, such that the collar 110 is seated on the nasal spray device with a spray outlet end of the nasal spray tip 12 exposed outside of the collar. The user may insert the nasal spray tip 12 in their nostril, until the shoulder 120 of the nasal spray guide is seated against a nasal surface (e.g., nasal base) of the user, thereby guiding an appropriate insertion depth for the nasal spray tip 12. A user may align an alignment indicator on the nasal spray guide with an anatomical landmark and/or the angle of the shoulder 120 may help guide the orientation of the nasal spray tip toward a predetermined orientation, thereby causing the nasal spray tip to target an intended tissue location (e.g., lateral turbinate). For example, in an example variation the nasal spray guide may include an alignment indicator that the user orients toward their septum, and the user may aim the outlet of the nasal spray tip toward their lateral turbinate. Once the nasal spray device is positioned at the appropriate depth and/or angle, the user may actuate the nasal spray device to administer fluid from the nasal spray device. The same procedure may be repeated on the opposite side in the opposite nostril if needed. Following administration of the fluid, the user may disinfect the nasal spray tip if desired

(e.g., with an alcohol wipe) and cover the nasal spray tip with the cap 150 of the nasal spray guide. Alternatively, the nasal spray guide may be removed from the nasal spray device and discarded.

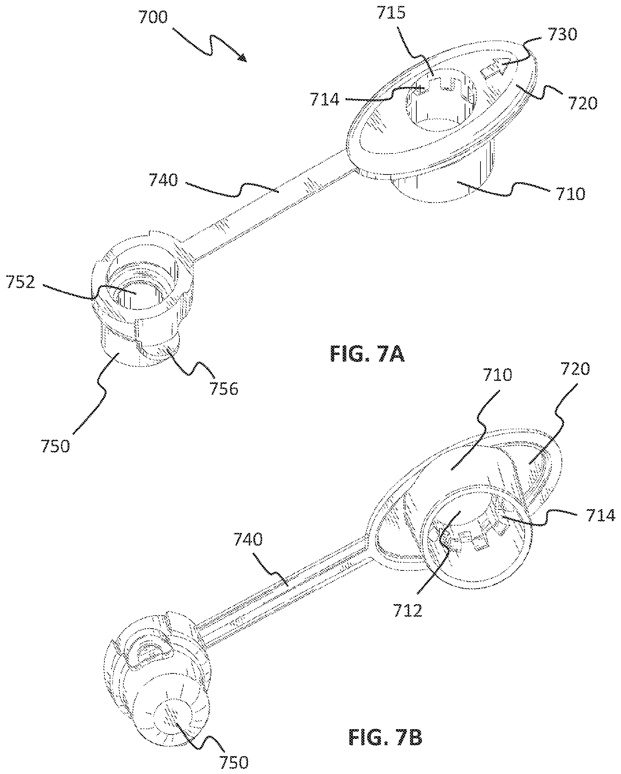

FIGS. 7A and 7B depict upper and lower perspective schematic views of an example variation of a nasal spray guide 700. Generally, the nasal spray guide 700 is similar to the nasal spray guide 100 described above, with further features as described below. As shown in FIG. 7A, the nasal spray guide 700 may include a collar 710 including a lumen 712, and a shoulder 720 comprising a shoulder surface extending radially from the lumen 712. The lumen 712 may be configured to receive a nasal spray tip of a nasal spray pump or other device (e.g., receive the nasal spray tip entirely therethrough such that a distal nozzle end of the nasal spray tip is exposed above the shoulder 720.

The collar 710 may be configured to be placed around the nasal spray tip of a nasal spray device, such as above an actuatable pump of the nasal spray device. The collar 710 may have a generally circular cross-sectional shape, with a generally circular lumen 710 positioned in the center of the collar 710. However, in other variations the collar and/or lumen may have other suitable cross-sectional shapes. The lumen 712 includes one or more circumferentially-distributed projections 714 that extend radially inward. As shown in FIGS 7A and 7B, the projections 714 (which may be interconnected by a ring 715) may narrow the diameter of the lumen 712 toward an end of the collar 710 that is proximate the shoulder 720, thereby helping to configure the lumen 712 to be sized to fit to a tapered nasal spray tip.

As shown in FIGS. 7A and 7B, the shoulder 720 may include an elliptical surface and be arranged on one end of the collar surrounding the lumen 712, though in other variations the shoulder 720 may have other suitable shapes (e.g., rectangular, etc.). The shoulder 720 may have rounded or beveled edges (e.g., to help reduce risk of injury when skin contacts the shoulder 720). In some variations, the shoulder 720 may be integrally formed with the collar 710 (e.g., via injection molding), though in other variations the collar 710 and the shoulder 720 may be separately formed and coupled together (e.g., with epoxy, interlocking features, one or more fasteners, etc.).

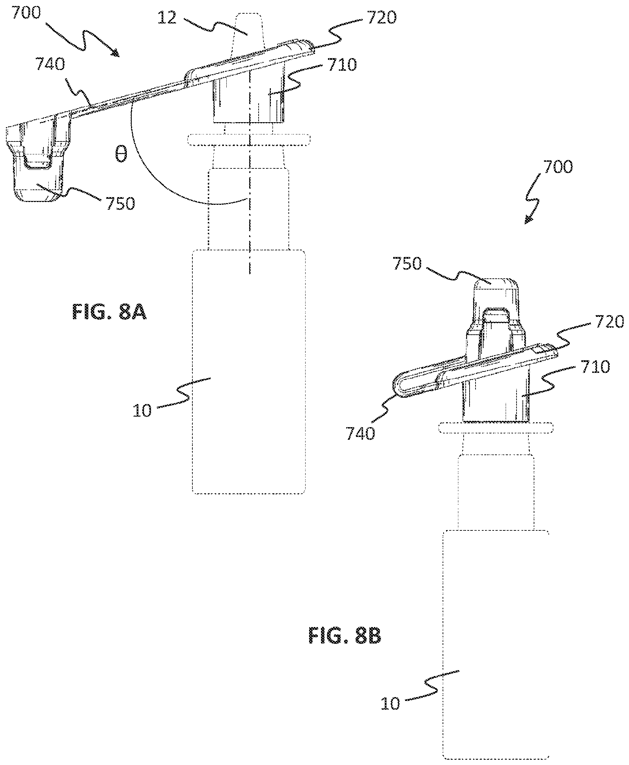

As shown in FIG. 8A, the elliptical surface of the shoulder 720 (the surface that is intended to be placed against a nasal surface of a user) may be obliquely angled relative to the lumen 112 of the collar 710 at an angle θ. The angle θ may, for example, be between about 60 degrees and about 85 degrees, or about 75 degrees.

The nasal spray guide 700 may further include a cap 750 with a cavity 752. The cap 750 and/or cavity 752 may have a stepped profile, with two or more tiers of progressively narrowing diameter terminating in a closed cap end. Furthermore, the cap 750 may include one or more gripping tabs 756 that provide an outwardly extending surface that a user may contact to manipulate the cap 750. For example, the cap 750 may include one gripping tab 756, two gripping tabs 756 on opposing sides of the cap 750, or more gripping tabs. An open end of the cap 752 may have a surface that is similarly angled at angle θ, so as to form a substantial seal when placed against the elliptical surface of the shoulder 720 (FIG. 8B).

As shown in FIGS. 8A and 8B, the cap 750 may be attached to the shoulder 720 with a tether 740. Additionally or alternatively, the cap may be attached to the collar 710 with the same tether or another tether. The tether 740 is depicted as a flexible rectangular member, though the tether 740 may have any suitable form (e.g., string-like member) to connect the cap 750 to the rest of the nasal spray guide. The tether 740 may be integrally formed with the cap 750, collar 710, and/or shoulder 720, or may be separately formed from and coupled to at least one of the cap, collar, and shoulder (e.g., with epoxy, interlocking features, one or more fasteners, etc.). The tether may be flexible such that the cap 750 may be moved between an uncapped configuration (FIG. 8A) that leaves a nasal spray tip 12 exposed, and a capped configuration (FIG. 8B) that covers the nasal spray tip 12 received within the cavity 752.

As best shown in FIG. 7A, in some variations, the nasal spray guide 720 may include an alignment indicator 730. In some variations, the alignment indicator 730 may be positioned on a side of the shoulder 720 intended to be adjacent to the nasal septum when the combination of the nasal spray guide 700 and the nasal spray device is used to administer fluid. The alignment indicator 730 is depicted in FIG. 7A as an arrow that is integrally formed in (e.g., molded into) the shoulder surface. However, other variations and locations of the alignment indicator 730 are possible, similar to the alignment indicator 130 described above.

Use of the nasal spray guide 700 is substantially similar to that described above for nasal spray guide 100. For example, FIG. 8A depicts a configuration of the nasal spray guide 700 arranged on a nasal spray device 10 with the distal end of the nasal spray tip 12 extending exposed beyond the collar 710 and shoulder 730. In this configuration, the cap 750 is disengaged from the shoulder 720, and a user may orient the nasal spray device such that the alignment indicator 730 points toward their septum, then insert the nasal spray tip 12 into their nostril until the shoulder 720 abuts a nasal surface of the user. The user may then actuate the nasal spray device to administer fluid from the nasal spray device. Thereafter, the user may remove the nasal spray tip 12 from their nostril, repeat the procedure on their other nostril (if desired or appropriate), then cover the nasal spray tip 12 with the cap 750 (or remove the nasal spray guide, such as for discard or storage).

### Examples

### Exemplary nasal spray guides

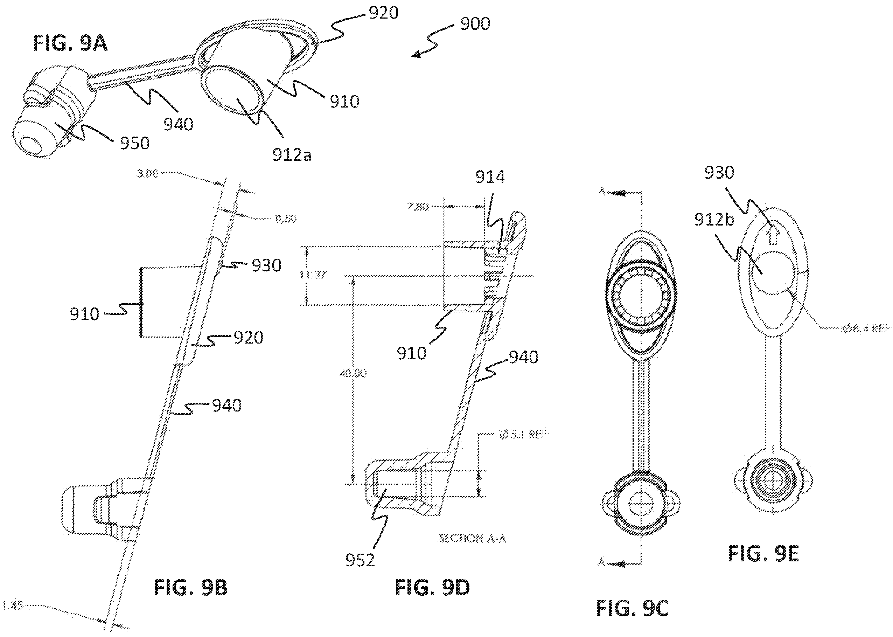

FIGS. 9A-9E depict various illustrative schematic views of an example variation of a nasal spray guide 900 that is substantially similar to nasal spray guide 700 shown in FIGS. 7A-7B and FIGS. 8A-8B, designed for use with an Aptar^{®} CPS (Cartridge Pump System) pump-type nasal spray. For example, nasal spray guide 900 includes a collar 910 with a lumen, a shoulder 920 extending radially outward from the collar 910, and a cap 950 that is attached to the shoulder 920 by a tether 940. The collar 910 has a lumen including a first, wider lumen portion 912a and narrower lumen portion 912b whose diameter is defined by the distance between opposite radial projections 914 within the lumen. A surface of the shoulder 920 further includes an arrow-shaped alignment indicator 930. All dimensions in FIGS. 9A-9E are expressed in millimeters.

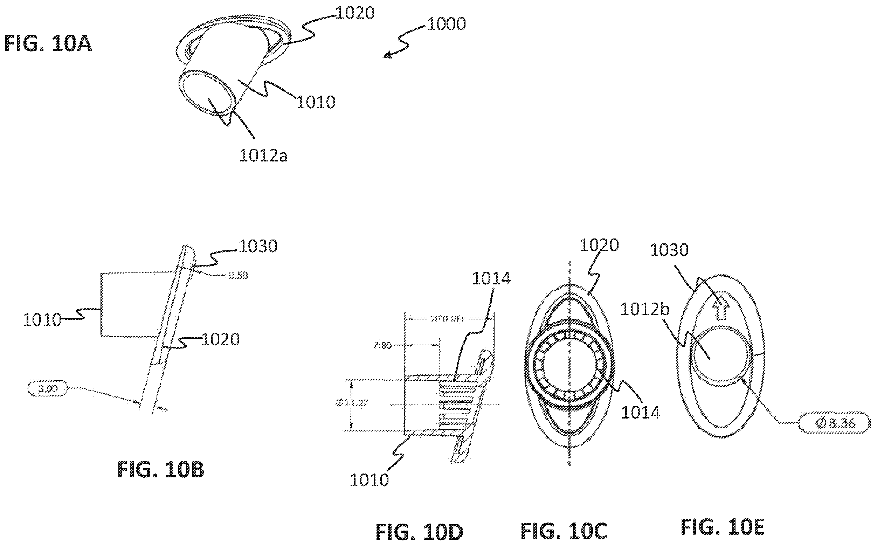

FIGS. 10A-10E depict various illustrative schematic views of another example variation of a nasal spray guide 1000 that is similar to nasal spray guide 900 shown in FIGS. 9A-9E, designed for use with an Aptar^{®} CPS nasal spray, except with a longer collar and without a cap or a tether. For example, nasal spray guide 1000 includes a collar 1010 with a lumen, a shoulder 1020 extending radially outward from the collar 1010. The collar 1010 has a lumen including a first, wider lumen portion 1012a and narrower lumen portion 1012b whose diameter is defined by the distance between opposite radial projections 1014 within the lumen. A surface of the shoulder 1020 further includes an arrow-shaped alignment indicator 1030. All dimensions in FIGS. 10A-10E are expressed in millimeters.

Whereas nasal spray guide 900 as shown in FIGS. 9A-9E and nasal spray guide 1000 as shown in FIGS. 10A-10E were designed for use with a particular nasal spray model, it will be appreciated that other embodiments of the nasal spray guide as described herein may be designed as appropriate for use with other nasal sprays having other form factors, and which are configured as a pump-type spray or with a propellant-type spray.

### Spray characterization studies

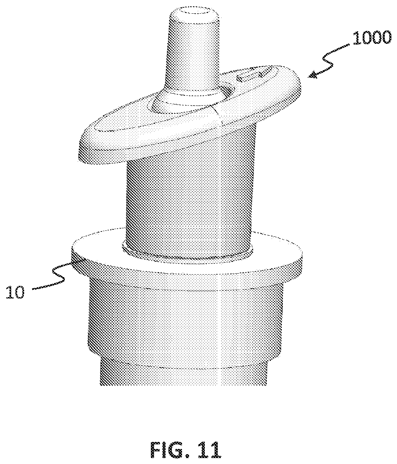

In certain variations, the nasal spray guide may be configured so that, when placed over the nozzle of a nasal spray pump, the nasal spray guide does not inhibit the spray nozzle orifice, or influence the spray functionality of the pump, for example pump delivery, spray pattern, droplet size distribution, and plume geometry. Spray characterization studies were conducted to study the effect of an embodiment of the nasal spray guide on spray formation characteristics of a nasal spray. The spray formation characteristics studied included pump delivery (PD), actuation force (AF), spray pattern (SP), droplet size distribution (DSD) and plume geometry (PG). As shown in Fig. 11, an embodiment of the nasal spray guide 1000 depicted in FIGS. 10A-10E was placed on an Aptar^{®} CPS nasal spray comprising a TYRVAYA^{™} composition, and the nasal spray was actuated under standard usage conditions. As detailed below, a comparison of the spray formation characteristics with and without the nasal spray guide confirmed that the placement of the nasal spray guide did not affect the spray formation characteristics of the nasal spray.

The pump delivery (PD) was measures as milligrams (mg) of the therapeutic composition ejected out of the nasal spray per actuation event. The results of the comparison are shown in Table 1 (n=10 for each condition). As shown in the table, there was no substantive difference in PD between nasal sprays with and without the nasal spray guide.

**Table 1: pump delivery**

| **Sample** | **7^{th} Prime (mg)** | **BOL 1 (mg)** | **BOL 2 (mg)** | **EOL 1 (mg)** | **EOL 2 (mg)** |
|---|---|---|---|---|---|
| Control | Mean 49.7 | Mean = 46.7 | Mean = 47.8 | Mean = 50.9 | Mean = 51.4 |
| | Range 47-52 | Range 43-53 | Range 45-52 | Range 48-53 | Range 48-55 |
| Nasal Spray Guide | Mean = 48.7 | Mean = 47.8 | Mean = 47.6 | Mean = 49.9 | Mean = 51.0 |
| | Range 44-52 | Range 43-54 | Range 43-54 | Range 44-54 | Range 48-54 |

| | | | | | |
|---|---|---|---|---|---|
| *7^{th} Prime = 7^{th} of 7 "priming" actuations to get pump ready for use; BOL = "beginning of life": first and second spray after priming; EOL= "end of life": 59^{th} and 60^{th} sprays when bottle is almost empty. | | | | | |

The actuation force (AF) was measures as kilograms (kg) of force generated by the nasal spray per actuation event. The results of the comparison are shown in Table 2 (n=10 for each condition). As shown in the table, there was no substantive difference in AF between nasal sprays with and without the nasal spray guide.

**Table 2: actuation force**

| **Sample** | **7^{th} Prime** | **BOL 1 (kg)** | **BOL 2 (kg)** | **EOL 1 (kg)** | **EOL 2 (kg)** |
|---|---|---|---|---|---|
| Control | | Mean = 3.7 | Mean = 3.8 | Mean = 3.8 | Mean = 3.7 |
| | | Range 3.6-4.1 | Range 3.5-4.2 | Range 3.5-4.0 | Range 3.5-4.2 |
| Nasal Guide | | Mean = 3.7 | Mean = 3.7 | Mean = 3.8 | Mean = 3.7 |
| | | Range 3.6-4.0 | Range 3.5-3.9 | Range 3.6-4.1 | Range 3.5-3.9 |

The spray pattern (SP) was measures as Dmax (diameter of largest drop in spray), ovality, and shape of the spray generated by the nasal spray per actuation event. The results of the comparison are shown in Table 3 (n=10 for each condition). As shown in the table, there was no substantive difference in SP between nasal sprays with and without the nasal spray guide.

**Table 3: spray pattern**

| **Sample** | **Mean Results** | **Range** |
|---|---|---|
| Control | Mean Dmax: 22.9 mm | Dmax: 19.77-26.62 mm |
| | Mean Ovality: 1.42 | Std Dev: 1.56 |
| | Shape: Circular or ellipsoidal of uniform density | % RSD: 6.83 |
| | | Ovality: 1.264-1.584 mm |
| | | Std Dev: 0.09 |
| | | % RSD: 6.58 |
| Nasal Spray Guide | Mean Dmax: 20.62 mm | Dmax: 18.69 - 22.95 |
| | Mean Ovality: 1.40 | Std Dev: 1.24 |
| | Shape: Circular or ellipsoidal of uniform density | % RSD: 6.02 |
| | | Ovality: 1.213-1.544 mm |
| | | Std Dev: 0.09 |
| | | % RSD: 6.71 |

The droplet size distribution (DSD) generated by the nasal spray per actuation event was also compared. The results of the comparison are shown in Table 4 (n=10 for each condition). The mean Dv values provide a measure of the DSD of the spray particles. "Mean Dv10" refers to spray particle diameter that is greater than 10% of all the spray particles in an actuation event. "Mean Dv50" refers to spray particle diameter that is greater than 50% of all the spray particles in an actuation event. "Mean Dv90" refers to spray particle diameter that is greater than 90% of all the spray particles in an actuation event. As shown in the table, there was no substantive difference in DSD between nasal sprays with and without the nasal spray guide.

**Table 4: droplet size distribution**

| **Sample** | **Mean Results** |
|---|---|
| Control | Mean Dv(10): 26.27 mm |
| | Mean Dv(50): 63.94 mm |
| | Mean Dv(90): 132.99 mm |
| | Mean %<10 mm: 0.21 |
| | Mean Span: 1.67 |
| Nasal Spray Guide | Mean Dv(10): 27.64 mm |
| | Mean Dv(50): 70.11 mm |
| | Mean Dv(90): 144.77 mm |
| | Mean %<10 mm: 0.17 |
| | Mean Span: 1.67 |

The plume geometry (PG) of the plume generated by the nasal spray per actuation event was also compared. The results of the comparison are shown in Table 5 (n=10 for each condition). As shown in the table, there was no substantive difference in PG between nasal sprays with and without the nasal spray guide.

**Table 5: plume geometry**

| **Sample** | **Mean** | **Range** |
|---|---|---|
| Control | Spray Angle: 36.51 | Spray Angle: 30.0 - 44.5 |
| | Plume Width: 39.83 | Plume Width: 32.16 - 49.47 |
| Nasal Guide | Spray Angle: 37.21 | Spray Angle: 31.4 - 43.5 |
| | Plume Width: 40.54 | Plume Width: 33.81 - 47.82 |

The foregoing description, for purposes of explanation, used specific nomenclature to provide a thorough understanding of the invention. However, it will be apparent to one skilled in the art that specific details are not required in order to practice the invention. Thus, the foregoing descriptions of specific embodiments of the invention are presented for purposes of illustration and description. They are not intended to be exhaustive or to limit the invention to the precise forms disclosed; obviously, many modifications and variations are possible in view of the above teachings. The embodiments were chosen and described in order to explain the principles of the invention and its practical applications, they thereby enable others skilled in the art to utilize the invention and various embodiments with various modifications as are suited to the particular use contemplated. It is intended that the following claims define the scope of the invention.

## Claims

1. A nasal spray guide (100), comprising:
a collar (110) defining a lumen (112) configured to receive a nasal spray tip; and
a shoulder (120) extending radially outward from the collar (110), wherein the shoulder (120) comprises a shoulder surface (122) angled relative to the lumen (112); **characterized in that** an inner surface of the collar (110) comprises one or more radially inward projections (114).

2. The guide of claim 1, further comprising a cap defining a cavity and configured to engage the shoulder (120) such that the cavity and the lumen (112) are substantially aligned.

3. The guide of claim 1, wherein the shoulder surface (122) is obliquely angled relative to the lumen (112).

4. The guide of claim 1, wherein the shoulder surface (122) is angled relative to the lumen (112) at an angle between about 60 degrees and about 80 degrees.

5. The guide of claim 1, wherein the shoulder surface (122) substantially surrounds the lumen (112).

6. The guide of claim 1, wherein the shoulder surface (122) is substantially planar.

7. The guide of claim 1, wherein the shoulder surface (122) is substantially planar, substantially surrounds the lumen (112), and is obliquely angled relative to the lumen (112).

8. The guide of claim 7, wherein the shoulder surface (122) is obliquely angled relative to the lumen (112) at an angle θ between about 60 degrees and about 80 degrees.

9. The guide of claim 1, wherein the shoulder surface (122) is substantially elliptical.

10. The guide of claim 2, wherein the cap is coupled to at least one of the collar (110) and the shoulder (120).

11. The guide of claim 10, wherein the cap is coupled to at least one of the collar (110) and the shoulder (120) by a tether

12. The guide of claim 11, wherein the tether comprises a flexible member.

13. The guide of claim 1, further comprising an alignment indicator.

14. The guide of claim 13, wherein the alignment indicator comprises an arrow on the shoulder (120).

15. The guide of claim 1, wherein the guide is constructed from a thermoplastic polymer, preferably wherein the thermoplastic polymer comprises an antimicrobial agent.

## Patentansprüche

1. Nasensprayführung (100), umfassend:
eine Manschette (110), die ein Lumen (112) definiert, das ausgebildet ist, um eine Nasensprühspitze aufzunehmen; und
eine Schulter (120), die sich radial von der Manschette (110) nach außen erstreckt, wobei die Schulter (120) eine Schulterfläche (122) umfasst, die relativ zu dem Lumen (112) geneigt ist; **dadurch gekennzeichnet, dass** eine Innenfläche der Manschette (110) einen oder mehrere radial nach innen gerichtete Vorsprünge (114) umfasst.

2. Führung nach Anspruch 1, die weiter eine Kappe umfasst, die einen Hohlraum definiert und ausgebildet ist, um mit der Schulter (120) in Eingriff zu stehen, sodass der Hohlraum und das Lumen (112) im Wesentlichen ausgerichtet sind.

3. Führung nach Anspruch 1, wobei die Schulterfläche (122) schräg relativ zu dem Lumen (112) geneigt ist.

4. Führung nach Anspruch 1, wobei die Schulterfläche (122) relativ zu dem Lumen (112) bei einem Winkel zwischen etwa 60 Grad und etwa 80 Grad geneigt ist.

5. Führung nach Anspruch 1, wobei die Schulterfläche (122) das Lumen (112) im Wesentlichen umschließt.

6. Führung nach Anspruch 1, wobei die Schulterfläche (122) im Wesentlichen eben ist.

7. Führung nach Anspruch 1, wobei die Schulterfläche (122) im Wesentlichen eben ist, das Lumen (112) im Wesentlichen umschließt und schräg relativ zu dem Lumen (112) geneigt ist.

8. Führung nach Anspruch 7, wobei die Schulterfläche (122) schräg relativ zu dem Lumen (112) bei einem Winkel θ zwischen etwa 60 Grad und etwa 80 Grad geneigt ist.

9. Führung nach Anspruch 1, wobei die Schulterfläche (122) im Wesentlichen elliptisch ist.

10. Führung nach Anspruch 2, wobei die Kappe mit mindestens einem von der Manschette (110) und der Schulter (120) gekoppelt ist.

11. Führung nach Anspruch 10, wobei die Kappe mittels einer Halteleine mit mindestens einem von der Manschette (110) und der Schulter (120) gekoppelt ist

12. Führung nach Anspruch 11, wobei die Halteleine ein flexibles Element umfasst.

13. Führung nach Anspruch 1, die weiter eine Ausrichtungsanzeige umfasst.

14. Führung nach Anspruch 13, wobei die Ausrichtungsanzeige einen Pfeil auf der Schulter (120) umfasst.

15. Führung nach Anspruch 1, wobei die Führung aus einem thermoplastischen Polymer hergestellt ist, wobei das thermoplastische Polymer bevorzugt einen antimikrobiellen Wirkstoff umfasst.

## Revendications

1. Guide de pulvérisation nasale (100), comprenant :
un collier (110) définissant une lumière (112) configurée pour recevoir un embout de pulvérisation nasale ; et
un épaulement (120) s'étendant radialement vers l'extérieur à partir du collier (110), dans lequel l'épaulement (120) comprend une surface d'épaulement (122) inclinée par rapport à la lumière (112) ;
**caractérisé en ce qu'**une surface interne du collier (110) comprend une ou plusieurs saillies s'étendant radialement vers l'intérieur (114).

2. Guide selon la revendication 1, comprenant en outre un capuchon définissant une cavité et configuré pour venir en prise avec l'épaulement (120) de telle sorte que la cavité et la lumière (112) sont sensiblement alignées.

3. Guide selon la revendication 1, dans lequel la surface d'épaulement (122) est inclinée de manière oblique par rapport à la lumière (112).

4. Guide selon la revendication 1, dans lequel la surface d'épaulement (122) est inclinée par rapport à la lumière (112) à un angle compris entre environ 60 degrés et environ 80 degrés.

5. Guide selon la revendication 1, dans lequel la surface d'épaulement (122) entoure sensiblement la lumière (112).

6. Guide selon la revendication 1, dans lequel la surface d'épaulement (122) est sensiblement plane.

7. Guide selon la revendication 1, dans lequel la surface d'épaulement (122) est sensiblement plane, entoure sensiblement la lumière (112), et est inclinée de manière oblique par rapport à la lumière (112).

8. Guide selon la revendication 7, dans lequel la surface d'épaulement (122) est inclinée de manière oblique par rapport à la lumière (112) à un angle Θ compris entre environ 60 degrés et environ 80 degrés.

9. Guide selon la revendication 1, dans lequel la surface d'épaulement (122) est sensiblement elliptique.

10. Guide selon la revendication 2, dans lequel le capuchon est couplé à au moins un élément parmi le collier (110) et l'épaulement (120).

11. Guide selon la revendication 10, dans lequel le capuchon est couplé à au moins un élément parmi le collier (110) et l'épaulement (120) par un lien.

12. Guide selon la revendication 11, dans lequel le lien comprend un organe flexible.

13. Guide selon la revendication 1, comprenant en outre un indicateur d'alignement.

14. Guide selon la revendication 13, dans lequel l'indicateur d'alignement comprend une flèche sur l'épaulement (120).

15. Guide selon la revendication 1, dans lequel le guide est construit à partir d'un polymère thermoplastique, de préférence dans lequel le polymère thermoplastique comprend un agent antimicrobien.
